# EUROPÄISCHE PATENTSCHRIFT

(11) **EP 2 589 350 B1**
(45) Veröffentlichungstag und Bekanntmachung des Hinweises auf die Patenterteilung: **15.04.2015**
(21) Anmeldenummer: 12183744.7
(22) Anmeldetag: 10.09.2012
(51) Int. Cl.: A61B 17/70

(54) **Dynamische Stabilisierungseinrichtung für Knochen**
Dynamic stabilisation device for bones
Dispositif de stabilisation dynamique pour les os

(30) Priorität: 05.11.2011 DE 102011055079
(43) Veröffentlichungstag der Anmeldung: 08.05.2013
(73) Patentinhaber: Universitätsmedizin der Johannes Gutenberg-Universität Mainz, 55131 Mainz (DE)
(72) Erfinder: Kantelhardt, Sven, 55129 Mainz (DE)
(74) Vertreter: Müller, Jochen

(56) Entgegenhaltungen:
- US-A1- 2005 085 814
- US-A1- 2007 100 341
- US-A1- 2009 093 820
- US-A1- 2009 234 388
- US-A1- 2010 211 104

## Beschreibung

Die Erfindung bezieht sich auf eine dynamische Stabilisierungseinrichtung für Knochen, insbesondere für Wirbel, mit zwei zueinander beabstandeten Knochenverankerungselementen, die über Befestigungselemente mit einem zumindest abschnittweise elastischen Stab verbindbar sind der eine verstellbare Einrichtung zur Beeinflussung seiner Elastizität aufweist, die zur Veränderung der freien Länge eines elastischen Abschnitts des Stabes ausgebildet ist , wobei die Einrichtung einen über den Stab verlagerbaren Schlitten umfasst, der den Stab umfangsseitig zumindest abschnittsweise umgreift.

Eine derartige Stabilisierungseinrichtung ist aus der US 2009/0093820 A1 bekannt.

Eine Stabilisierungseinrichtung mit den Merkmalen des Oberbegriffs des Anspruchs 1 ist aus der US 2005/085814 A1 bekannt.

Im Rahmen von Wirbelsäulenverletzungen (Trauma), Abnutzungen (Degeneration) und anderen Erkrankungen (Tumoren etc.) kommt es immer wieder zu Instabilitäten der Wirbelsäule. Gemäß einer klassischen Methode erfolgt in solchen Fällen eine Stabilisierung des betroffenen Wirbelsäulensegmentes mit Pedikelschrauben hinten zur Zuggurtung der hinteren Säule und Platzhaltern im Bandscheibenfach zur Abstützung der ventralen Säulen.

Nach einer Weiterentwicklung standen so genannte dynamische Systeme zur Verfügung, die dem Knochen einen höheren Reiz zur Knochenheilung bzw. -neubildung zu geben, da keine vollständig starre Stabilisierung erfolgt. Laboruntersuchungen zeigten ein schnelleres Heilen der Knochen bei einer nicht vollständigen chirurgischen Fusion. Bei den dynamischen Systemen zur Stabilisierung eines Wirbelsäulensegmentes, wird zwischen den Pedikelschrauben ein flexibler Verbindungsstab angeordnet. Nach einer weiteren Methodenverbesserung erfolgte ein Abstützen einer geschädigten Bandscheibe durch die ausschließliche Verwendung von mittels eines flexiblen Verbindungsstabes miteinander verbundener Pedikelschrauben. Durch diese Methode ist der operative Eingriff und die Veränderung der normalen Anatomie erheblich kleiner als bei den früheren Behandlungsmethoden. Außerdem ist es möglich die bekannten Techniken als "topping off" oder "topping down" zu kombinieren, so dass ein geschädigtes Segment fusioniert wird, das Nachbarsegment, welches weniger intensive Abnutzungen zeigt aber nur dynamisch stabilisiert wird. Dies erscheint insbesondere auch daher sinnvoll zu sein, da bei einer Fusion eines Segmentes die Nachbarsegmente einen Beweglichkeitsverlust ausgleichen und daher ihre Belastung steigt. Beispielsweise wird bei einem "topping off", das untere Segment mit Pedikelschrauben und Platzhaltern in der Bandscheibe versorgt, das obere Segment nur mit dem dynamischen Schraube-Stab-System.

Aus der Praxis sind eine Vielzahl von dynamischen Systemen zur Stabilisierung von Segmenten der Wirbelsäule bekannt, die beispielsweise flexible Verbindungsstäbe aus Polyetheretherketon (PEEK) oder aus Titanfedern umfassen oder im Wesentlichen starre Verbindungsstäbe, die mit Gelenken an den Pedikelschrauben befestigt werden.

Die bekannten Systeme zur dynamischen Stabilisierung sind insofern problematisch, als eine zu "weiche" Einstellung, insbesondere ein verhältnismäßig flexibler Verbindungsstab, im Hinblick auf die Stabilisierung nicht hilfreich ist und eine zu "harte" Einstellung, also beispielsweise ein zu steifer Verbindungsstab, zu einer Lockerung der Pedikelschrauben oder einem Brechen des Systems führt.

Eine optimale Steifigkeit des Systems, vorzugsweise des Verbindungsstabes, für alle Patienten ist nicht erzielbar, da schwerere Patienten mit einem festen Knochen sicherlich ein steiferes System zur wirksamen Stützung der betreffenden Segmente benötigen, als ein Patient mit Osteoporose, der eine relativ "weiche" Einstellung, also ein flexibles System, benötigt, um eine Lockerung der Pedikelschrauben im Knochen zu verhindern. Darüber hinaus ist damit zu rechnen, dass sich die "optimale" Festigkeit" des Systems im Laufe des Lebens eines Patienten verändert, nicht nur mit zunehmender Osteoporose oder zunehmendem Gewicht, sondern auch in Abhängigkeit der Krankheitsentwicklung und des Einheilens der Pedikelschrauben. So ist z. B. direkt nach Implantation möglicherweise ein relativ flexibles, also weich eingestelltes, System günstig, um das Einheilen zu erleichtern, wohingegen mit zunehmender Abheilung und unter Umständen einer beginnenden spontanen Fusion der Bandscheibe eine immer "härtere" Einstellung notwendig ist.

Die DE 10 2006 003 374 A1 offenbart ein flexibles Verbindungselement für die Implantation in den menschlichen oder tierischen Körper, insbesondere für die Stabilisierung der Wirbelsäule, mit einem Stab, der mindestens einen flexiblen Bereich und mindestens zwei Verbindungsbereiche zur Befestigung zu verbindender Elemente aufweist. Zumindest in einem Teil des flexiblen Bereichs des Stabes ist ein flexibles Element oder Stabilisierungselement vorgesehen, das außerhalb des Stabquerschnitts angeordnet ist und mindestens an einer Stelle mit dem Stab axial unverschiebbar verbunden ist. Dieses dynamische Stabilisierungssystem ermöglicht die Kombination verschiedener modularer Komponenten vor bzw. während der Implantation. Eine individuelle Einstellung der Festigkeit bzw. Vorspannung des Implantates ist nicht vorgesehen.

Im Weiteren zeigt die DE 102 36 691 A1 eine dynamische Stabilisierungseinrichtung für Knochen, insbesondere für Wirbel, mit wenigstens einem ersten und einem zweiten Knochenverankerungselement, mit jeweils einem ersten, in einem Knochen zu verankernden Abschnitt und einem zweiten, mit einem Stab zu verbindenden Abschnitt, und einem die Knochenverankerungselemente verbindenden Stab, wobei die Knochenverankerungselemente mit dem Stab wahlweise fest oder in Richtung der Stabachse verschiebbar verbindbar sind. Zwischen den Knochenverankerungselementen ist ein in Richtung der Stabachse elastisch vorspannbares Element angeordnet. Das elastische Element bewirkt in erster Linie eine mehr oder weniger starke Distraktion, im Sinne eines Auseinanderdrückens der Wirbelkörper und hat keinen Einfluss auf die Flexibilität bzw. Elastizität des Stabes zwischen den Verankerungsschrauben, d. h. die mögliche Durchbiegung des Stabes wird durch das elastische Element nicht beeinflusst.

Der Erfindung liegt die Aufgabe zugrunde, eine dynamische Stabilisierungseinrichtung für Knochen der eingangs genannten Art zu schaffen, die individuell an patientenspezifische Erfordernisse anpassbar und dabei einfach handhabbar ist.

Erfindungsgemäß wird die Aufgabe durch die Merkmale des Anspruchs 1 gelöst.

Die Unteransprüche stellen vorteilhafte Ausgestaltungen der Erfindung dar.

Eine dynamische Stabilisierungseinrichtung für Knochen, insbesondere für Wirbel, umfasst zwei zueinander beabstandete Knochenverankerungselemente, die über Befestigungselemente mit einem zumindest abschnittweise elastischen Stab verbindbar sind, der eine verstellbare Einrichtung zur Beeinflussung seiner Elastizität aufweist, die zur Veränderung der freien Länge eines elastischen Abschnitts des Stabes ausgebildet ist. Die Einrichtung umfasst einen über den Stab verlagerbaren Schlitten, der den Stab umfangsseitig zumindest abschnittsweise umgreift, wobei der Stab zumindest eine sich parallel zu seiner Längsachse erstreckende Führungsnut aufweist, in die eine Führungsnase des verschiebbaren Schlittens gleitend eingreift, wobei sich die Führungsnut über den von dem Schlitten überdeckbaren elastischen Abschnitt erstreckt und eine derartige Länge aufweist, dass der Schlitten in seiner einen Endlage den elastischen Abschnitt freigibt und in seiner anderen Endlage den elastischen Abschnitt über dessen Länge überdeckt.

Der elastische, also selbsttätig aus einer verformten Lage in eine Ursprungsposition zurückstellbare, Stab kann durch die stufenlos verstellbare Einrichtung individuell an die patientenspezifischen Belange angepasst werden, in dem seine Elastizität verändert wird. Beispielsweise wird der Stab in einer "weichen", also relativ elastischen Einstellung bei einem Patienten mit Osteoporose verwendet, wobei eine verhältnismäßig große Elastizität vorzugsweise eine Lockerung der insbesondere als Pedikelschrauben ausgebildeten Knochenverankerungselemente im Knochen verhindert. Bei einem Patienten mit einem festen Knochen und einem verhältnismäßig hohen Gewicht lässt sich die Einrichtung derart verstellen, dass die Elastizität des Stabes verringert wird, also ein relativ steifes System zur wirksamen Stützung der Knochen zur Verfügung steht.

Weist der elastische Abschnitt eine relativ kurze freie Länge auf, dann ist der Stab "hart" eingestellt, ist der elastische Abschnitt verhältnismäßig lang, dann sind geringe Kräfte zur Verformung des Stabes erforderlich und der Stab ist "weich" eingestellt.

Der über den Stab verlagerbare Schlitten, der den Stab umfangsseitig zumindest abschnittsweise umgreift, wird mehr oder weniger über den elastischen Bereich des Stabes geschoben, um dessen Elastizität zu verändern. Hierbei wirkt der Schlitten wie eine den Stab in seinem elastischen Bereich stützende Schiene. Durch die Führungsnut befindet sich der Schlitten in einer sowohl zu dem Stab als auch zu einem Stellrad definierten Lage.

Zweckmäßigerweise ist die Einrichtung vor und/oder nach der Implantation der Stabilisierungseinrichtung einstellbar. Die stufenlose Einstellbarkeit der Einrichtung nach der Implantation ist von besonderem Interesse, da aufgrund eines Heilungsprozesses oder sich ändernder patientenbezogener Anforderungen eine Veränderung der Elastizität des Stabes vorteilhaft ist. Die Einrichtung lässt sich bei der Implantation derart platzieren, dass sie mittels Magnetkraft oder nach einem kleinen Eingriff, insbesondere einer Punktion, einstellbar ist.

Zur Feinverstellung des Schlittens weist die Einrichtung ein Stellrad zur Verschiebung des Schlittens auf, wobei das Stellrad mit einem Eingriff für ein Werkzeug versehen und/oder aus einem magnetischen Werkstoff gefertigt sowie, insbesondere in dem Stab, drehbar gelagert ist. Das beispielsweise mit einem Schraubendreher, einem Innensechskant- oder Sechsrundschlüssel, der unter dem Markennamen Torx bekannt ist, verdrehbare Stellrad beaufschlagt den Schlitten zu dessen stufenloser linearer Verschiebung gegenüber dem Stab kraft- oder formschlüssig. Wenn das Stellrad aus einem magnetischen Werkstoff besteht, ist seine Verdrehung mittels Magnetkraft nach der Implantation ohne einen chirurgischen Eingriff möglich. Zweckmäßigerweise weist das Stellrad an zumindest einem Teil seines äußeren Umfangs eine Verzahnung auf, die mit einer korrespondierenden Zahnstange des zu dem Stellrad verschiebbaren Schlittens zusammenwirkt. Die Zahnstange kann selbstverständlich integraler Bestandteil des Schlittens sein, wonach eine Verzahnung in einem Randbereich des Schlittens eingearbeitet ist.

Nach einer Weiterbildung weist der Stab zur Bildung seines elastischen Abschnitts eine Querschnittsschwächung auf, die durch den Schlitten überdeckbar ist. Die Reduzierung des Querschnitts muss nicht gleichmäßig über den Umfang verteilt erfolgen, sondern kann einseitig vorgesehen sein, so dass der Stab asymmetrisch ist. Der geschwächte Querschnitt des Stabes wird zu den zu stabilisierenden Knochen ausgerichtet, um eine erwünschte Bewegung in einer bestimmten Richtung zu ermöglichen.

Damit ein Einwachsen des Schlittens nach der Implantation verhindert und seine Verlagerung entlang der Längsachse des Stabes sichergestellt ist, ist der Stab unter Freilassung von Ansätzen für die Befestigungselemente mit einem Mantel aus einem Kunststoff überzogen, wobei der Mantel insbesondere die Einrichtung und den elastischen Abschnitt des Stabes überdeckt.

Für eine Verstellung des Schlittens nach der Implantation mittels eines an dem Stellrad angreifenden Werkzeuges ist ein freier Zugang zu dem einen Eingriff des Stellrades für ein Werkzeug ohne eine wesentliche Beschädigung des Mantels erforderlich, daher weist der Mantel umfangsseitig eine konische Bohrung auf, die koaxial zu dem Stellrad verläuft. Zweckmäßigerweise ist der Mantel aus einem Silikon oder Polyetheretherketon (PEEK) gefertigt. Selbstverständlich sind dem Fachmann weitere biokompatible Werkstoffe bekannt, die vorliegend Verwendung finden können.

Der Stab ist aus einem Titan-Werkstoff gefertigt oder in Längsrichtung zweiteilig ausgeführt, wobei das eine Teil aus einem Titan-Werkstoff und das andere Teil aus einem Kunststoff, insbesondere einem Polyetheretherketon-Komposit, besteht.

Damit der Schlitten den Stab in seiner Elastizität begrenzt, ist der Schlitten aus einem Titan-Werkstoff gefertigt, der vorzugsweise eine gegenüber dem Titan-Werkstoff des Stabes höhere Festigkeit aufweist.

Es versteht sich, dass die vorstehend genannten und nachstehend noch zu erläuternden Merkmale nicht nur in der jeweils angegebenen Kombination, sondern auch in anderen Kombinationen verwendbar sind. Der Rahmen der Erfindung ist nur durch die Ansprüche definiert.

Die Erfindung wird im Folgenden anhand zweier Ausführungsbeispiele unter Bezugnahme auf die zugehörige Zeichnung näher erläutert. Es zeigt:
- Fig. 1: eine schematische Darstellung einer in zwei Knochen verankerten dynamischen Stabilisierungseinrichtung,
- Fig. 2: eine vergrößerte Teildarstellung einer Einzelheit II gemäß Fig. 1 von oben mit einem Schlitten in einer ersten Endlage,
- Fig. 3: eine Teildarstellung der Stabilisierungseinrichtung nach Fig. 1 von der Seite,
- Fig. 4: eine Seitenansicht der Darstellung nach Fig. 1,
- Fig. 5: eine Darstellung nach Fig. 3 mit dem Schlitten in einer zweiten Endlage,
- Fig. 6: eine Draufsicht auf die Darstellung nach Fig. 4,
- Fig. 7: eine weitere Darstellung der Einzelheit II gemäß Fig. 1,
- Fig. 8: eine Darstellung eines Schnittes entlang der Linie VII-VII nach Fig. 5,
- Fig. 9: eine Darstellung eines Schnittes entlang der Linie VIII-VIII nach Fig. 5,
- Fig. 10: eine Darstellung eines Schnittes entlang der Linie IX-IX nach Fig. 5,
- Fig. 11: eine Darstellung eines Schnittes entlang der Linie VIII-VIII nach Fig. 5 durch einen Stab in alternativer Ausgestaltung und
- Fig. 12: eine Seitenansicht des Schlittens.

Die dynamische Stabilisierungseinrichtung 1 umfasst als Pedikelschrauben ausgeführte Knochenverankerungselemente 2, die beabstandet zueinander in zwei gegeneinander zu stabilisierende Knochen 3, vorliegend Wirbel, befestigt sind. An ihren über die Knochen 3 vorstehenden Enden sind die Knochenverankerungselemente 2 mit Befestigungselementen 4 zur Halterung eines zumindest abschnittweise elastischen Stabes 5 versehen, dem unter einem Mantel 6 aus einem Kunststoffmaterial, beispielsweise einem Silikon oder Polyetheretherketon (PEEK), eine verstellbare Einrichtung 7 zur Beeinflussung seiner Elastizität zugeordnet ist. Der Mantel 6 verhindert ein Einwachsen der Einrichtung 7 nach der Implantation und gewährleistet demzufolge deren Verstellbarkeit.

Der Stab 5 weist einen im Wesentlichen D-förmigen Querschnitt mit einer abgeflachten Seite 8, von der beidseitig parallel zueinander verlaufende Schenkel 9 abgehen, die über eine der abgeflachten Seite 8 gegenüberliegende Krümmung 10 miteinander verbunden sind. Zur Ausbildung eines Abschnitts 11 mit einer gegenüber dem übrigen Verlauf des Stabes 5 erhöhten Elastizität verläuft eine querschnittsreduzierende Ausfräsung 12 von der abgeflachten Seite 8 auch über eine Mittelachse des Stabes 5 erstreckend in Richtung der Krümmung 10, wobei der wirksame Querschnitt des Stabes 5 in etwa in dessen Mitte am geringsten bemessen ist. In die Schenkel 9 des Stabes 5 sind parallel zu der abgeflachten Seite 8 und zur Längsachse des Stabes 5 ausgerichtete Führungsnuten 13 eingearbeitet, die eine Länge aufweisen, die mindestens der doppelten Länge der Ausfräsung 12 entspricht, wobei sich die Führungsnuten 13 beiderseits über die Mitte des Stabes 5 erstrecken.

In der abgeflachten Seite 8 des Stabes 5 befindet sich eine Aussparung, in die ein Stellrad 14 eingesetzt ist, dessen Oberseite 15 bündig mit der abgeflachten Seite 8 des Stabes 5 verläuft und an dessen Unterseite zentrisch eine in dem Stab 5 drehbar gelagerte Achse 16 angeformt ist. In die Oberseite 15 des Stellrades 14 ist ein Eingriff 17 für ein Werkzeug zur Verdrehung des Stellrades 14 eingelassen. Korrespondierend zu dem Eingriff 17 weist der Mantel 6 eine konische Bohrung 25 für das Werkzeug auf. Das Stellrad 14 weist an seinem peripheren Umfang eine Verzahnung 18 auf, die mit einer korrespondierenden Zahnstange 19 eines zu dem Stellrad 14 verschiebbaren Schlittens 20 zusammenwirkt.

Der im Querschnitt im Wesentlichen U-förmige Schlitten 20 ist auf dem Stab 5 durch eine Drehung des Stellrades 14 stufenlos verschiebbar gelagert, wobei sein U-Bogen 21 zu der Krümmung 10 des Stabes 5 korrespondiert und seine U-Schenkel 22 parallel zu den Schenkeln 9 des Stabes verlaufen. Die U-Schenkel 22 sind mit Führungsnasen 23 versehen, die in die Führungsnuten 13 des Stabes 5 eingreifen. An den freien Enden der U-Schenkel 22 sind zueinander ausgerichtete Ansätze 24 vorgesehen, von denen der eine als die mit dem Stellrad 14 zusammenwirkende Zahnstange 19 ausgebildet ist. Die Ansätze 24 sind in Vertiefungen 27 in der abgeflachten Seite 8 des Stabes 5 verschiebbar derart gelagert, dass ihre freie Oberseite bündig mit der abgeflachten Seite 8 des Stabes 5 verläuft.

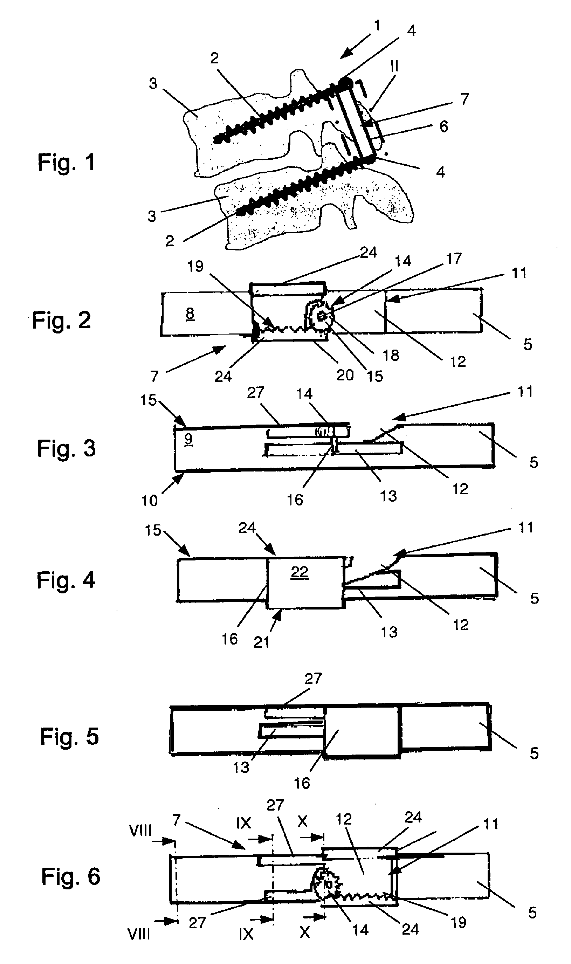

In einer ersten Endlage gemäß den Fig. 2 und 4 gibt der Schlitten 5 den elastischen Abschnitt des Stabes 5 vollkommen frei, d. h. er befindet sich komplett neben der Ausfräsung 12 in einem Bereich, in dem der Stab 5 keine Querschnittsreduzierung aufweist. Da der reduzierte Querschnitt des Stabes 5 völlig freiliegt, weist der Stab seine größtmögliche Elastizität auf. Er ist also bereits unter der Einwirkung relativ geringer Kräfte verformbar. Um die Elastizität des Stabes 5 zu verringern, also seine Steifigkeit zu erhöhen, wird der Schlitten in Richtung der Ausfräsung 12 in eine beliebige Lage verschoben, indem das Stellrad 14 mittels eines in den Eingriff 17 eingesetzten Werkzeuges verdreht wird. Hierbei überdeckt der Schlitten 20 mit seinen U-Schenkeln 22 seitlich die Ausfräsung 12 und stützt den Stab 5 im Bereich seiner Krümmung 10. Da der Schlitten 20 über seine Führungsnasen 23 und Ansätze 24 an dem Stab 5 gehalten ist, kann er entsprechenden Kräften bzw. Drehmomenten, die auf dem Stab 5 wirken, entgegen wirken. Nach einer weiterergehenden Verkürzung der freien Länge des elastischen Abschnitts durch eine entsprechende Verschiebung des Schlittens 20 gelangt dieser in seine zweite Endlage gemäß den Fig. 5 und 6, in der er die Ausfräsung 12 dreiseitig auf deren gesamter Länge überdeckt.

Ist die Stabilisierungseinrichtung 1 implantiert und die Elastizität des Stabes 5 muss verändert werden, ist es erforderlich, das Stellrad 14 in entsprechender Richtung zu verdrehen. Die Verdrehung kann beispielsweise mittels Magnetkräften vorgenommen werden, wenn das Stellrad aus einem entsprechenden magnetischen Werkstoff besteht oder das Stellrad 14 wird, beispielsweise mittels eines bildgebenden Verfahrens, lokalisiert und die entsprechende Stelle punktiert, um ein Werkzeug durch die Bohrung 25 des Mantels 6 in den Eingriff 17 des Stellrades 14 zu führen, um das Stellrad 14 zu verdrehen.

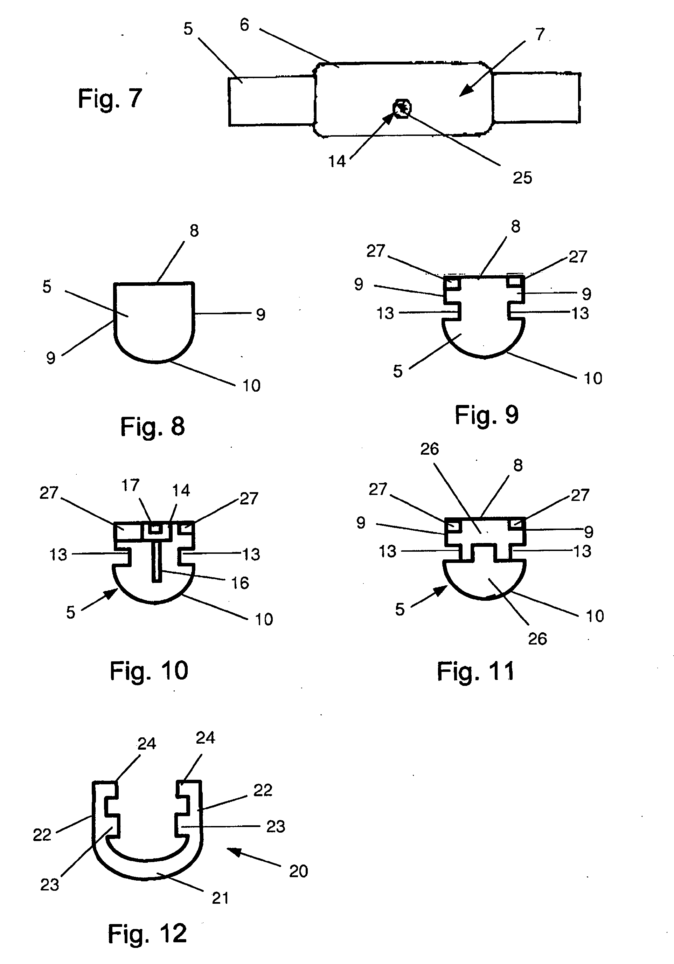

Der Stab 5 und der Schlitten 20 sind aus einem Titan-Werkstoff gefertigt. Gemäß Fig. 11 ist der Stab 5 in Längsrichtung zweiteilig ausgeführt, wobei die beiden Teile 26 aus unterschiedlichen Werkstoffen gefertigt sein können, zum Beispiel das eine Teil 26 aus einem Titan-Werkstoff und das andere Teil 26 aus einem Kunststoff, insbesondere einem Polyetheretherketon-Komposit.

### Bezugszeichenliste

- 1.: Stabilisierungseinrichtung
- 2.: Knochenverankerungselement
- 3.: Knochen
- 4.: Befestigungselement
- 5.: Stab
- 6.: Mantel
- 7.: Einrichtung
- 8.: Seite von 5
- 9.: Schenkel von 5
- 10.: Krümmung von 5
- 11.: elast. Abschnitt
- 12.: Ausfräsung
- 13.: Führungsnut
- 14.: Stellrad
- 15.: Oberseite
- 16.: Achse
- 17.: Eingriff
- 18.: Verzahnung
- 19.: Zahnstange
- 20.: Schlitten
- 21.: U-Bogen
- 22.: U-Schenkel
- 23.: Führungsnase
- 24.: Ansatz
- 25.: Bohrung
- 26.: Teil von 5
- 27.: Vertiefung

## Patentansprüche

1. Dynamische Stabilisierungseinrichtung (1) für Knochen (3), insbesondere für Wirbel, mit zwei zueinander beabstandeten Knochenverankerungselementen (2), die über Befestigungselemente (4) mit einem zumindest abschnittweise elastischen Stab (5) verbindbar sind, der eine verstellbare Einrichtung (7) zur Beeinflussung seiner Elastizität aufweist, die zur Veränderung der freien Länge eines elastischen Abschnitts (11) des Stabes (5) ausgebildet ist, wobei die Einrichtung (7) einen über den Stab (5) verlagerbaren Schlitten (20) umfasst, der den Stab (5) umfangsseitig zumindest abschnittsweise umgreift, **dadurch gekennzeichnet, dass** der Stab (5) zumindest eine sich parallel zu seiner Längsachse erstreckende Führungsnut (13) aufweist, in die eine Führungsnase (23) des verschiebbaren Schlittens (20) gleitend eingreift, wobei sich die Führungsnut (13) über den von dem Schlitten (20) überdeckbaren elastischen Abschnitt (11) erstreckt und eine derartige Länge aufweist, dass der Schlitten (20) in seiner einen Endlage den elastischen Abschnitt (11) freigibt und in seiner anderen Endlage den elastischen Abschnitt (11) über dessen Länge überdeckt.

2. Stabilisierungseinrichtung nach Anspruch 1, **dadurch gekennzeichnet, dass** die Einrichtung (7) vor und/oder nach der Implantation der Stabilisierungseinrichtung (1) einstellbar ist.

3. Stabilisierungseinrichtung nach Anspruch 1 oder 2, **dadurch gekennzeichnet, dass** die Einrichtung (7) ein Stellrad (14) zur Verschiebung des Schlittens (20) aufweist, wobei das Stellrad (14) mit einem Eingriff (17) für ein Werkzeug versehen und/oder aus einem magnetischen Werkstoff gefertigt und, insbesondere in dem Stab (5), drehbar gelagert ist.

4. Stabilisierungseinrichtung nach Anspruch 3, **dadurch gekennzeichnet, dass** das Stellrad (14) an zumindest einem Teil seines äußeren Umfangs eine Verzahnung (18) aufweist, die mit einer korrespondierenden Zahnstange (19) des zu dem Stellrad (14) verschiebbaren Schlittens (20) zusammenwirkt.

5. Stabilisierungseinrichtung nach Anspruch 1, **dadurch gekennzeichnet, dass** der Stab (5) zur Bildung seines elastischen Abschnitts (11) eine Querschnittsschwächung aufweist, die durch den Schlitten (20) überdeckbar ist.

6. Stabilisierungseinrichtung nach einem der Ansprüche 1 bis 5, **dadurch gekennzeichnet, dass** der Stab (5) unter Freilassung von Ansätzen für die Befestigungselemente (4) mit einem Mantel (6) aus einem Kunststoff überzogen ist.

7. Stabilisierungseinrichtung nach Anspruch 6, **dadurch gekennzeichnet, dass** der Mantel (6) die Einrichtung (7) und den elastischen Abschnitt (11) des Stabes (5) überdeckt.

8. Stabilisierungseinrichtung nach Anspruch 6 oder 7, **dadurch gekennzeichnet, dass** der Mantel (6) umfangsseitig eine konische Bohrung (25) aufweist, die koaxial zu dem Stellrad (14) verläuft.

9. Stabilisierungseinrichtung nach einem der Ansprüche 6 bis 8, **dadurch gekennzeichnet, dass** der Mantel (6) aus einem Silikon oder Polyetheretherketon (PEEK) gefertigt ist.

10. Stabilisierungseinrichtung nach einem der Ansprüche 1 bis 6, **dadurch gekennzeichnet, dass** der Stab (5) aus einem Titan-Werkstoff gefertigt oder in Längsrichtung zweiteilig ausgeführt ist, wobei das eine Teil (26) aus einem Titan-Werkstoff und das andere Teil (26) aus einem Kunststoff, insbesondere einem Polyetheretherketon-Komposit, besteht.

11. Stabilisierungseinrichtung nach einem der Ansprüche 1 bis 5, **dadurch gekennzeichnet, dass** der Schlitten (20) aus einem Titan-Werkstoff gefertigt ist.

12. Stabilisierungseinrichtung nach Anspruch 11, **dadurch gekennzeichnet, dass**, der Titan-Werkstoff des Schlittens (20) eine gegenüber dem Titan-Werkstoff des Stabes (5) höhere Festigkeit aufweist.

13. Stab zur Verwendung in einer dynamischen Stabilisierungseinrichtung (1) nach einem der Ansprüche 1 bis 12

## Claims

1. Dynamic stabilization device (1) for bones (3), in particular for vertebrae, with two bone-anchoring elements (2) spaced apart from each other and connectable, via fastening elements (4), to a rod (5) which is elastic at least in part and which has an adjustable device (7) for influencing the elasticity of the rod (5), which device (7) is designed for changing the free length of an elastic portion (11) of the rod (5), the device (7) comprising a carriage (20) which is moveable over the rod (5) and which engages at least in part about the circumference of the rod (5), **characterized in that** the rod (5) has at least one guide groove (13) which extends parallel to the longitudinal axis thereof and in which a guide lug (23) of the moveable carriage (20) slidingly engages, wherein the guide groove (13) extends over the elastic portion (11) coverable by the carriage (20) and is of such a length that the carriage (20), in one end position, releases the elastic portion (11) and, in its other end position, covers the elastic portion (11) over the length thereof.

2. Stabilization device according to Claim 1, **characterized in that** the device (7) is adjustable before and/or after the implantation of the stabilization device (1).

3. Stabilization device according to Claim 1 or 2, **characterized in that** the device (7) has an adjusting wheel (14) for moving the carriage (20), wherein the adjusting wheel (14) is provided with a socket (17) for a tool and/or is made of a magnetic material and is mounted rotatably, in particular in the rod (5).

4. Stabilization device according to Claim 3, **characterized in that** the adjusting wheel (14) has, on at least part of its outer circumference, a toothing (18) which cooperates with a corresponding toothed rack (19) of the carriage (20) that is moveable to the adjusting wheel (14).

5. Stabilization device according to Claim 1, **characterized in that** the rod (5) has a cross-sectional narrowing, which forms its elastic portion (11) and is coverable by the carriage (20).

6. Stabilization device according to one of Claims 1 to 5, **characterized in that** the rod (5) is covered with a coating (6) of a plastic, leaving projections free for the fastening elements (4).

7. Stabilization device according to Claim 6, **characterized in that** the coating (6) covers the device (7) and the elastic portion (11) of the rod (5).

8. Stabilization device according to Claim 6 or 7, **characterized in that** the coating (6) has on its circumference a conical bore (25), which extends coaxially with respect to the adjusting wheel (14).

9. Stabilization device according to one of Claims 6 to 8, **characterized in that** the coating (6) is made of a silicone or polyether ether ketone (PEEK).

10. Stabilization device according to one of Claims 1 to 6, **characterized in that** the rod (5) is made of a titanium material or is configured in two parts in the longitudinal direction, wherein one part (26) is made of a titanium material and the other part (26) is made of a plastic, in particular a polyether ether ketone composite.

11. Stabilization device according to one of Claims 1 to 5, **characterized in that** the carriage (20) is made of a titanium material.

12. Stabilization device according to Claim 11, **characterized in that** the titanium material of the carriage (20) has a greater strength than the titanium material of the rod (5).

13. Rod for use in a dynamic stabilization device (1) according to one of Claims 1 to 12.

## Revendications

1. Dispositif de stabilisation dynamique (1) pour des os (3), en particulier pour des vertèbres, comportant deux éléments d'ancrage d'os (2) espacés l'un de l'autre, qui peuvent être assemblés par des éléments de fixation (4) à une barre au moins localement élastique (5), qui présente un dispositif réglable (7) pour influencer son élasticité, lequel est conçu en vue de l'ancrage de la longueur libre d'une section élastique (11) de la barre (5), dans lequel le dispositif (7) comprend un coulisseau (20) déplaçable sur la barre (5) qui entoure la barre (5) en périphérie au moins localement, **caractérisé en ce que** la barre (5) présente au moins une rainure de guidage (13) s'étendant parallèlement à son axe longitudinal, dans laquelle est engagé un ergot de guidage glissant (23) du coulisseau déplaçable (20), dans lequel la rainure de guidage (13) s'étend sur la section élastique (11) pouvant être recouverte par le coulisseau (20) et présente une longueur telle que le coulisseau (20) libère dans sa première position d'extrémité la section élastique (11) et dans son autre position d'extrémité recouvre la section élastique (11) sur la longueur de celle-ci.

2. Dispositif de stabilisation selon la revendication 1, **caractérisé en ce que** le dispositif (7) peut être réglé avant et/ou après l'implantation du dispositif de stabilisation (1).

3. Dispositif de stabilisation selon la revendication 1 ou 2, **caractérisé en ce que** le dispositif (7) présente une roue de réglage (14) pour le déplacement du coulisseau (20), dans lequel la roue de réglage (14) est munie d'une prise (17) pour un outil et/ou est fabriquée en un matériau magnétique et est montée de façon rotative, en particulier dans la barre (5).

4. Dispositif de stabilisation selon la revendication 3, **caractérisé en ce que** la roue de réglage (14) présente sur au moins une partie de son pourtour une denture (18), qui coopère avec une crémaillère correspondante (19) du coulisseau (20) déplaçable vers la roue de réglage (14).

5. Dispositif de stabilisation selon la revendication 1, **caractérisé en ce que** la barre (5) présente, pour la formation de sa section élastique (11), une réduction de section transversale, qui peut être recouverte par le coulisseau (20).

6. Dispositif de stabilisation selon l'une quelconque des revendications 1 à 5, **caractérisé en ce que** la barre (5) est revêtue d'une enveloppe (6) en une matière plastique avec dégagement d'appuis pour les éléments de fixation (4).

7. Dispositif de stabilisation selon la revendication 6, **caractérisé en ce que** l'enveloppe (6) recouvre le dispositif (7) et la section élastique (11) de la barre (5).

8. Dispositif de stabilisation selon la revendication 6 ou 7, **caractérisé en ce que** l'enveloppe (6) présente en périphérie un perçage conique (25), qui s'étend coaxialement à la roue de réglage (14).

9. Dispositif de stabilisation selon l'une quelconque des revendications 6 à 8, **caractérisé en ce que** l'enveloppe (6) est fabriquée en un silicone ou en polyétheréthercétone (PEEK).

10. Dispositif de stabilisation selon l'une quelconque des revendications 1 à 6, **caractérisé en ce que** la barre (5) est fabriquée en un matériau de titane ou est réalisée en deux parties en direction longitudinale, dans lequel une première partie (26) se compose d'un matériau de titane et l'autre partie (26) se compose d'une matière plastique, en particulier d'un composite de polyétheréthercétone.

11. Dispositif de stabilisation selon l'une quelconque des revendications 1 à 5, **caractérisé en ce que** le coulisseau (20) est fabriqué en un matériau de titane.

12. Dispositif de stabilisation selon la revendication 11, **caractérisé en ce que** le matériau de titane du coulisseau (20) présente une résistance plus élevée que le matériau de titane de la barre (5).

13. Barre à utiliser dans un dispositif de stabilisation dynamique (1) selon l'une quelconque des revendications 1 à 12.
